Europäisches Patentamt

**European Patent Office**

**Office européen des brevets**

(11) Numéro de publication: **0 246 967 B1**

(12) **FASCICULE DE BREVET EUROPEEN**

(45) Date de publication de fascicule du brevet:
**15.01.92**

(51) Int. Cl.⁵: **C12P 21/02, A61K 37/02**

(21) Numéro de dépôt: **87401122.4**

(22) Date de dépôt: **19.05.87**

(54) **Nouvelle lymphokine supressive de l'activation plaquettaire, son procédé d'isolement et de purification et médicaments la contenant.**

(30) Priorité: **21.05.86 FR 8607194**

(43) Date de publication de la demande:
**25.11.87 Bulletin 87/48**

(45) Mention de la délivrance du brevet:
**15.01.92 Bulletin 92/03**

(84) Etats contractants désignés:
**AT BE CH DE ES FR GB GR IT LI LU NL SE**

(56) Documents cités:
**WO-A-85/00750**

**THE JOURNAL OF IMMUNOLOGY, vol. 118, no. 6, juin 1977, pages 2015-2019, The William & Wilkins Co., US; H.G. REMOLD et al.: "Two migration inhibitory factors with different chromatographic behavior and isoelectric points"**

**THE JOURNAL OF IMMUNOLOGY, vol. 131, no. 6, décembre 1983, pages 2848-2852, The American Association of Immunologists, US; T.M. AUNE et al.: "Purification and initial characterization of the lymphokine soluble immune response suppressor"**

(73) Titulaire: **INSTITUT PASTEUR DE LILLE**
**Rue du Professeur A. Calmette BP 245**
**F-59019 Lille Cédex(FR)**

Titulaire: **INSTITUT PASTEUR**
**28, rue du Docteur Roux**
**F-75724 Paris Cédex 15(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Capron, André**
**58 rue du Capitaine Jasmin**
**F-59133 Phalempin(FR)**
Inventeur: **Joseph, Michel**
**24 rue Desrousseaux**
**F-59800 Lille(FR)**
Inventeur: **Pancre, Véronique**
**57 rue Nicolas Leblanc**
**F-59000 Lille(FR)**
Inventeur: **Auriault, Claude**
**1 place de Verdun**
**F-59310 Mouchin(FR)**

NATURE, vol. 303, 30 juin 1983, pages 810-812, MacMillan Journals Ltd, GB; M. JO-SEPH et al.: "A new function for platelets: IgE-dependent killing of schistosomes"

(74) Mandataire: Ores, Irène et al
CABINET ORES 6, Avenue de Messine
F-75008 Paris(FR)

## Description

La présente invention est relative à une nouvelle lymphokine suppressive de l'activation plaquettaire, à son procédé d'isolement et de purification et aux médicaments la contenant.

L'on sait que les cellules T stimulées excrètent un certain nombre de facteurs solubles parmi lesquels certains sont capable de réguler les fonctions des cellules effecteurs.

Il a été démontré récemment [cf. M. JOSEPH, C. AURIAULT, A. CAPRON, M. VORNG et P. VIENS, NATURE 303 : 810] que les plaquettes sanguines prélevées chez des rats infectés par Schistosoma mansoni, expriment des propriétés antiparasites destructrices in vitro, qui augmentent au cours de l'infection. Le maximum de cytotoxicité a été observé lorsque les rats ont exprimé un taux élevé d'immunité vis-à-vis de la réinfection. Cette cytotoxicité plaquettaire décline rapidement après 8 semaines d'infection, en dépit de la présence d'anticorps IgE dans le sérum de ces rats infectés. C'est la raison pour laquelle les Inventeurs ont été amené à considérer que les facteurs des cellules T, produits après stimulation de cellules T suppresseurs par des antigènes circulants de S. Mansoni, jouent un rôle dans cette diminution, ce mécanisme pouvant apparaître comme une régulation en retour des fonctions immunes des plaquettes dans l'infection du rat. De même, il a été montré que les plaquettes humaines peuvent être induites en effecteurs cytotoxiques contre les schistosomules, par incubation avec du sérum riche en IgE de patients atteints de schistosomiase ou de patients allergiques asthmatiques, le processus cytotoxique étant retardé dans ce dernier cas par l'addition d'anti-IgE ou d'un allergène spécifique [cf. M. JOSEPH, J.C. AMEISEN, J.P. KUSNIERZ, V. PANCRÉ, M. CAPRON et A. CAPRON, 1984, C.R. ACAD. SC. PARIS 298 : 55].

La présente invention a pour but d'isoler un facteur suppresseur de l'activité des plaquettes sanguines, qui est capable de jouer, entre autres, un rôle d'immunomodulateur des manifestations allergiques.

La présente invention a pour objet un facteur obtenu à partir de cellules T stimulées par la Concanavaline A ou par un antigène, capable d'inhiber la cytotoxicité plaquettaire IgE-dépendante vis-à-vis de jeunes larves de S. mansoni, de diminuer fortement la chimioluminescence de plaquettes dans une réaction IgE/anti-IgE, qui est un correlat de la cytotoxicité antiparasitaire et d'inhiber l'activation plaquettaire dans des intolérances non-IgE dépendantes, lequel facteur est désigné sous le nom de lymphokine suppressive de l'activité plaquettaire (PASL) et est produit par les lymphocytes T OKT8[+].

Selon un mode de réalisation de la PASL, celle-ci est isolée de surnageants de cultures de lymphocytes T OKT8[+] stimulés par la Concanavaline A ou un antigène.

Selon un autre mode de réalisation de la PASL, dans le cas où elle est isolée de surnageants de cultures de lymphocytes T OKT8[+] stimulés par un antigène, les lymphocytes T ont été stimulés par des antigènes d'Echinococcus granulosus ou de Schistosoma mansoni.

La présente invention a également pour objet un procédé de préparation de la PASL qui consiste à stimuler des cellules T d'origine humaine ou murine par la Concanavaline A ou un stimulant mitogènique analogue ou par un antigène pris dans le groupe qui comprend les antigènes de S. mansoni et les antigènes d'Echinococcus granulosus, à cultiver les cellules T stimulées, pendant une durée appropriée, à récupérer les surnageants de culture et à les filtrer, éventuellement après centrifugation, à travers des membranes de dimension de pores de l'ordre de 0,22 $\mu$m, puis à les purifier par gelfiltration et à soumettre la fraction protéique active recueillie, préalablement identifiée par recherche de l'activité biologique ou immunologique et éventuellement lyophilisée, à une chromatographie en phase inverse sur un gel de silice greffé, de granulomètrie et de porosité calibrées, suivie d'une élution par un gradient de $CH_3CN-H_2O$ contenant 1% d'acide trifluoroacétique, allant de 1-99 à 60-40, la fraction protéique purifiée contenant la PASL étant ensuite avantageusement lyophilisée après détection par enregistrement de densité optique à 215 nm et détermination de l'activité biologique.

Selon un mode de mise en oeuvre avantageux du procédé d'obtention de la PASL conforme à la présente invention, l'activité biologique de la fraction protéique active et de la fraction protéique active purifiée contenant la PASL est déterminée par l'obtention de 60% d'inhibition de la cytotoxicité par le surnageant de culture de lymphocytes T stimulés par la Concanavaline A ou par des antigènes d'E. granulosus, vis-à-vis de plaquettes sanguines normales utilisées comme cellules effectrices, incubées avec du sérum de patients atteints de schistosomiase ou avec du sérum de patients allergiques présentant un taux élevé d'IgE circulantes et stimulés par des anti-IgE ou par l'allergène spécifique.

La présente invention a en outre pour objet un nouveau médicament immunomodulateur des manifestations allergiques qui est caractérisé en ce que son constituant actif est la PASL isolée de surnageants de cultures de lymphocytes T stimulés par un agent mitogène tel que la Concanavaline A ou analogue ou par un antigène approprié tel que notamment un antigène d'E. granulosus ou de S. mansoni, qui présente les propriétés énoncées plus haut et dont le poids moléculaire est de l'ordre de 15 à 20 kDa et le Pi est de l'ordre de 4,4 à 5,0, laquelle lymphokine possède une structure de liaison spécifique du type d'un récepteur, à la surface des plaquettes sanguines, c'est-à-dire une spécificité d'adsorption sur les plaquettes.

Les tests menés pour vérifier l'activité biologique du surnageant de cultures de lymphocytes T stimulés conformément à la présente invention ont permis de parvenir aux conclusions suivantes :

Effet du surnageant sur la cytotoxicité des plaquettes

Les surnageants obtenus après stimulation de lymphocytes T, périphériques ou d'amygdales, humains normaux, par la Concanavaline A (0,01 à 5 µg/ml) ou de lymphocytes T de rate d'un patient atteint d'Echinococcose, par des antigènes d'E. granulosus (5 à 100 µg/ml), testés dans des processus cytotoxiques de plaquettes vis-à-vis de schistosomules, ont permis d'obtenir les résultats réunis dans le Tableau I ci-après :

## TABLEAU I

### Effet de la PASL sur la cytotoxicité antiparasitaire IgE-dépendante de plaquettes sanguines murines et humaines provenant d'individus infectés et allergiques

| Cytotoxicité | Homme[a] | | Rat[b] |
|---|---|---|---|
| | A | B | |
| Dans un milieu activateur | 72,7± 2,9 | 81,7±5,5 | 71,2±5,2 |
| . avec du surnageant Con. A | 20,5± 2,1 | 22,1±4,0 | 19,5±2,5 |
| . avec du surnageant Ag | 35,0±15,0 | 33,6±6,1 | 21,5±0,5 |
| . avec du milieu de culture de lymphocytes | 70,2± 3,2 | 81,5±4,6 | 78,0±7,0 |

N.B. Dans tous les cas la cytotoxicité avec des sérums de rat ou humains normaux, n'excède pas 9,5 ±7,3.

a) Les plaquettes purifiées ont été incubées avec du sérum A (de patients atteints de Schistosomiase mansoni) ou B (de donneurs allergiques) en présence de larves de S. mansoni (Schistosomules). Le surnageant de lymphocytes ou le milieu de culture de lymphocytes et les anti-IgE ou l'allergène correspondant dans le cas d'incubation dans du sérum de donneurs allergiques, ont été ajoutés et la cytotoxicité a été appréciée en pourcentage de schistosomules

mortes à la fin d'une durée d'incubation de 24 heures à 37°C (moyenne ± écart type).

b) Les plaquettes purifiées ont été incubées avec du sérum de rats atteints de Schistosomiase mansoni en présence de larves de S. mansoni (schistosomules). Le surnageant de lymphocytes ou le milieu de culture de lymphocytes a été ajouté et la cytotoxicité a été appréciée en pourcentage de schistosomules mortes à la fin d'une durée d'incubation de 24 heures à 37°C (moyenne ± écart type).

Les Inventeurs ont mis en évidence que les surnageants sont actifs directement sur les fonctions plaquettaires et non par protection des cibles, car la préincubation séparée des plaquettes effectrices ou des schistosomules avec les surnageants de lymphocytes T, suivie d'un test de cytotoxicité dans lequel les plaquettes et les parasites ont été séparés par une membrane en polycarbonate de porosité 0,2 μ, ne détermine l'inhibition attendue que dans le cas où les plaquettes ont été préincubées et non dans le cas où les larves ont été incubées, ainsi que cela ressort du Tableau 2 qui va suivre.

## TABLEAU 2

### Action directe de la PASL sur les plaquettes sanguines humaines

|  | P/S | P*/S | P/S* |
|---|---|---|---|
| Cytotoxicité IgE/anti-IgE | 92,3±5,1 | 14,6±9,2 | 71,5±6,3 |

Les plaquettes (P) humaines et les schistosomules (S), traitées (*) ou non par du surnageant de lymphocytes, ont été séparées par un filtre (/) en polycarbonate de porosité 0,22 μm dans des chambres de Boyden.
La cytotoxicité a été appréciée en pourcentage de schistosomules mortes à la fin d'une période d'incubation de 24 heures à 37°C (moyenne I.S.D.).

De plus, les Inventeurs ont démontré que cette inhibition n'est pas la conséquence d'un effet toxique du surnageant de lymphocytes T car il n'a pas été détecté de LDH dans la suspension de plaquettes (Lactico-deshydrogénase).

La stimulation mitogénique de lymphocytes T de rate de rats soit par la Concanavaline A soit par le PHA [(2μg/ml); PHA = Phytohémagglutinine] ou la stimulation antigénique de lymphocytes T mésentériques ou de rate de rat infecté par S. mansoni, par de l'antigène de schistosome adulte, produit des surnageants qui inhibent la cytotoxicité IgE-dépendante de plaquettes de rats (40 à 60% d'inhibition), ce qui montre que les lymphocytes T de rats sécrètent, de même que les lymphocytes T humains, la lymphokine PASL qui est un facteur inhibiteur de plaquettes.

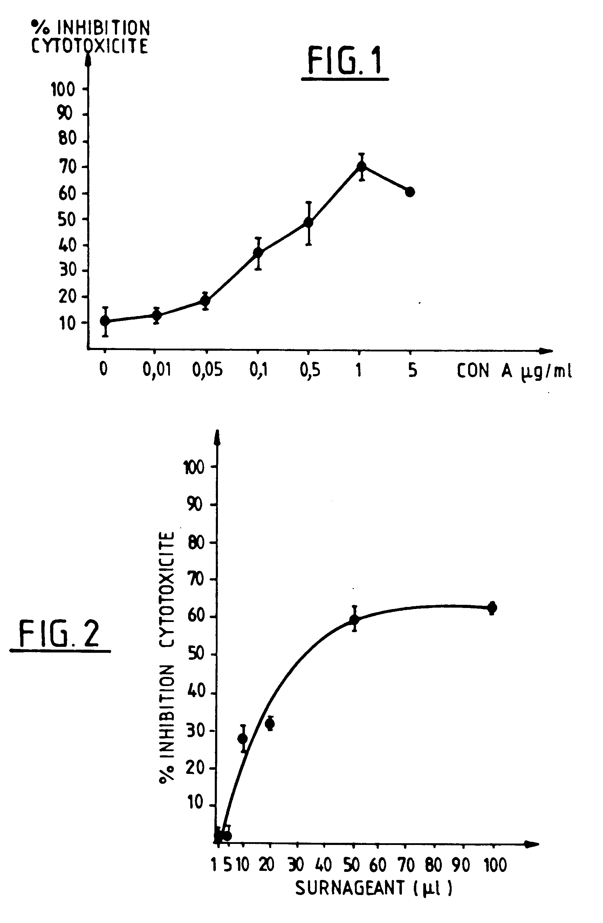

Comme le montre la Figure 1 annexée, qui représente le pourcentage d'inhibition de la cytotoxicité en fonction de la quantité de Concanavaline A (exprimée en μg/ml) utilisée comme stimulant, l'effet inhibiteur est proportionnel à la dose de Concanavaline A utilisée pour la stimulation des cellules T et l'effet optimal est obtenu à une dose finale de 1 μg/ml. Les surnageants produits en l'absence de Concanavaline A expriment également une activité suppressive, mais sensiblement inférieure à celle observée avec des surnageants activés à la lectine (20-25%).

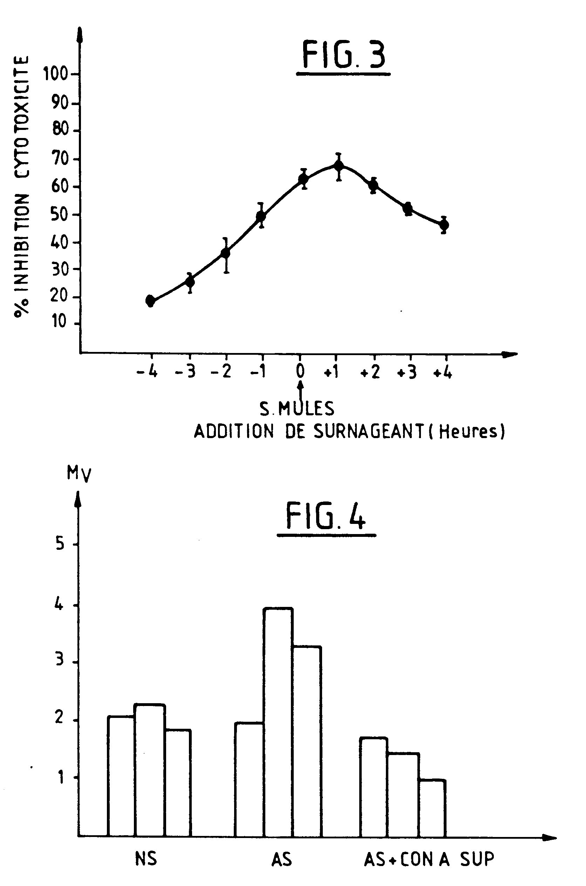

Comme le montre la Figure 2, qui représente le pourcentage d'inhibition de la cytotoxicité en fonction de la quantité de surnageant (exprimée en $\mu$l) mise en oeuvre, le surnageant agit d'une manière qui est fonction de la quantité mise en oeuvre, pour atteindre un plateau à 50 $\mu$l (1:4 de dilution finale), ce qui est la dose utilisée pour toutes les expériences ultérieures. L'effet de l'addition de surnageants à différentes périodes avant et après le commencement du processus cytotoxique est représenté à la Figure 3 annexée dans laquelle le pourcentage d'inhibition de la cytotoxicité est représenté en fonction de l'addition de surnageant dans le temps, exprimée en heures; l'inhibition optimale a été obtenue par addition du surnageant de cellules T au cours de la première heure de contact entre les plaquettes effectrices et les schistosomules (dans cette Figure "Smules" signifie schistosomules).

Structure de liaison sur les plaquettes

Trois incubations successives de surnageant de cellules T qui contient le facteur conforme à l'invention, qui est capable d'inhiber les propriétés destructrices des plaquettes vis-à-vis des schistosomules, avec des plaquettes mises en comprimés, suppriment son activité inhibitrice de la cytotoxicité des plaquettes : cette absorption du facteur suppresseur par les plaquettes suggère l'existence d'une structure de liaison. Par contre, lorsqu'on utilise des lignées cellulaires de myelome d'IgE, de K562, de U937 ou des macrophages broncho-alvéolaires, comme absorbants, l'effet inhibiteur du surnageant de lymphocytes est préservé.

Effet du surnageant sur la chimioluminescence des plaquettes

Les Inventeurs ont démontré précédemment [C. R. ACAD. SC. PARIS, 1984, 298:55 déja cité] que, dans la cytotoxicité IgE-dépendante vis-à-vis des schistosomules, il est possible d'obtenir la production de métabolites de l'oxygène par les plaquettes humaines, mesurée par chimioluminescence.

L'effet du surnageant de lymphocytes T stimulés par la Concanavaline A (1 $\mu$g/ml) sur la chimiolumi-nescence des plaquettes a été testé : comme le montre la Figure 4 annexée, une incubation de plaquettes avec du surnageant de lymphocytes T pendant une heure, à une dilution finale de 1:4, diminue fortement (de 60%) la production de métabolites de l'oxygène qui est normalement observée dans le cas où les plaquettes normales ont été stimulées par une réaction IgE/anti-IgE ou dans le cas où des plaquettes de patients allergiques ont été stimulées par l'allergène correspondant, ce qui confirme le rôle direct de la lymphokine conforme à l'invention, vis-à-vis des plaquettes elles-mêmes.

Dans la Figure 4, les initiales NS et AS ont les significations suivantes :

NS = incubées dans du sérum normal,

AS = incubées dans du sérum de patients allergiques,

AS + ConA Sup = incubées dans du sérum de patients allergiques et du surnageant de lymphocytes.

L'activité maximum de la chimioluminescence luminol/luciférine de $5.10^5$ plaquettes dans du PBS a été enregistrée dans les cinq minutes qui ont suivi l'addition du milieu (1ère colonne de chaque groupe) ou de l'agent retardateur :anti-IgE (10 $\mu$g d'anticorps/ml) (2ème colonne de chaque groupe) ou allergène (10 $\mu$g/ml) (3ème colonne de chaque groupe).

Caractérisation des cellules impliquées dans la production de lymphokine suppressive

Pour définir le type de cellules responsables de la production du facteur inhibiteur conforme à la présente invention, les Inventeurs ont examiné l'effet d'une déplétion de sous-population T sélective : ils on traité des cellules T par de l'anticorps monoclonal OKT4 (pour les sous-classes auxiliaire/inducteur) ou par de l'anticorps monoclonal OKT8 (pour les sous-classes supresseur/destructeur) en présence de complément. Les cellules ont alors été stimulées par la Concanavaline A et on a examiné si de la lymphokine suppressive a été produite.

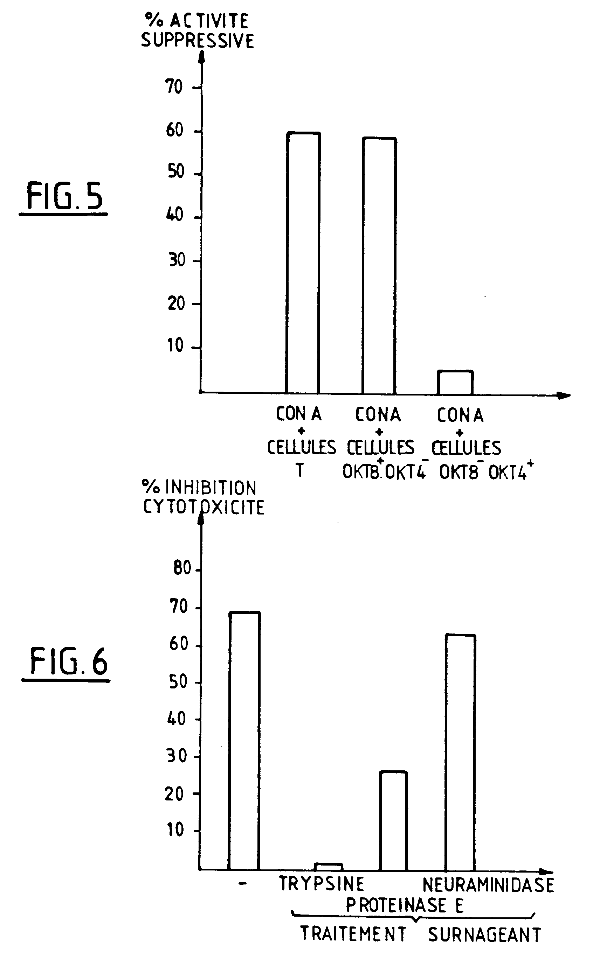

Comme le montre la Figure 5, la dépletion de lymphocytes OKT $8^+$ a supprimé la production du facteur inhibiteur, tandis que la dépletion de cellules OKT $4^+$ n'a pas modifié sa formation. La Figure 5 illustre le taux d'activité suppressive respective de PASL obtenue à partir de cellules T, de cellules OKT8$^+$OKT4$^-$ et de cellules OKT8$^-$OKT4$^+$, stimulées par la Concanavaline A. Les résultats sont exprimés en suppression % (moyenne) de la cytotoxicité IgE-dépendante, vis-à-vis des schistosomules.

Cette observation montre bien qu'une sous-population de cellules T suppressives produit le facteur responsable de l'inhibition observée des fonctions effectrices des plaquettes.

Effet du traitement de cellules T par l'indométhacine sur la production de la lymphokine (PASL) inhibitrice conforme à l'invention

Les prostaglandines jouent, comme on le sait, un rôle dans l'induction de cellules T suppressives non-spécifiques, pendant la stimulation mitogènique et antigènique de lymphocytes. Les Inventeurs ont donc traité les lymphocytes par l'indométhacine ($10^{-5}$ M concentration finale) qui est un inhibiteur de la synthèse des prostaglandines et ils ont examiné la production de lymphokine inhibitrice induite par l'agent mitogène. La production de ce facteur est demeurée inchangée dans ces conditions (48,0 ± 3,0% d'activité suppressive avec l'indométhacine, pour 45,0 ± 2,5% sans l'indométhacine).

Caractéristiques physicochimiques de la lymphokine inhibitrice plaquettaire (PASL) conforme à l'invention.

L'effet suppresseur est demeuré inchangé après dialyse du surnageant de lymphocytes pendant 24 heures contre une solution tampon de phosphate (PBS). Le facteur inhibiteur est stable vis-à-vis de la chaleur (56°C pendant une heure ou 100°C pendant 5 minutes) et des acides, car une dialyse pendant 24 heures contre un tampon à pH2 n'a pas altéré son effet suppresseur, ainsi que cela ressort du Tableau 3 ci-après qui montre les propriétés physicochimiques de la PASL : la stabilité de la PASL vis-à-vis de la chaleur a été évaluée en chauffant les surnageants au bain-marie à 56°C pendant 120 minutes, ou à 100°C pendant 5 à 10 minutes. La PASL dialysée a été préparée par dialyse du surnageant à 4°C pendant 24 heures contre du PBS, avec des changements multiples de milieu. Les surnageants traités par un acide ont été préparés par dialyse à 4°C pendant 24 heures contre du tampon glycine à pH2, suivie d'une dialyse pendant 24 heures supplémentaires contre du PBS pour éliminer l'acide en excès.

## TABLEAU 3
### Propriétés physicochimiques de la PASL

| Traitement | Indice d'inhibition % |
|---|---|
| – | 64,0±1,4 |
| A. Chauffage | |
| 120 minutes à 56°C | 65,7±5,6 |
| 5 minutes à 100°C | 74,8±8,8 |
| B. Dialyse pendant 24 heures | 64,3±3,6 |
| C. Traitement acide pendant 24 heures | 68,5±2,5 |

Comme le montre la Figure 6 annexée, qui montre le pourcentage d'inhibition de la cytotoxicité de surnageants traités par diverses enzymes, respectivement la trypsine, la protéase K et la neuraminidase, la lymphokine isolée conformément à la présente invention est sensible à la trypsine et à la protéase K, tandis que la neuraminidase n'a aucune influence sur son activité.

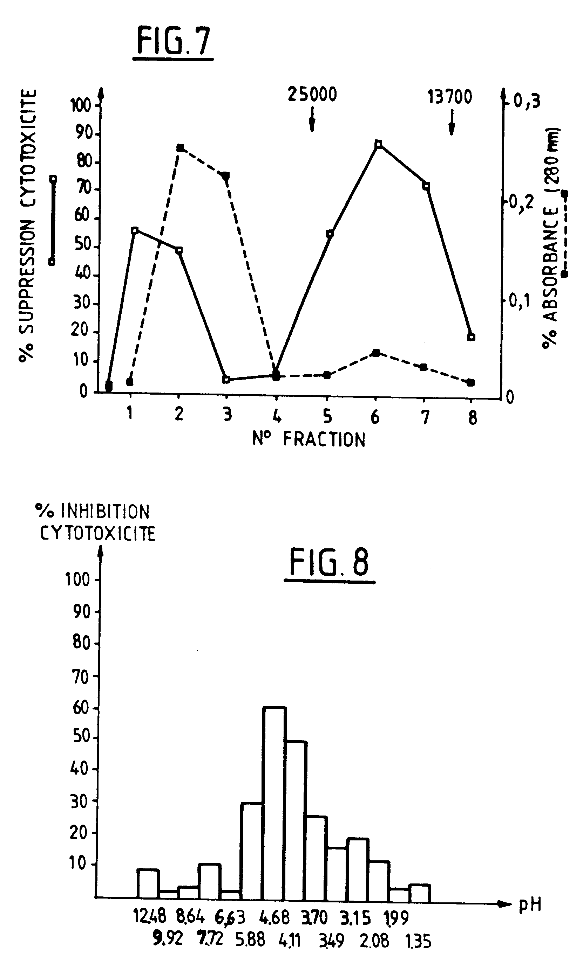

Un profil chromatographique de la lymphokine inhibitrice des plaquettes sanguines, conforme à la présente invention, sur un tamis moléculaire en Sephadex-G75, est représenté à la Figure 7. La PASL a été fractionnée sur une colonne de Sephadex-G75 préparée dans du tampon phosphate (pH 7,4). Les fractions éluées ont été testées pour leur activité PASL dans la cytotoxicité plaquettaire IgE-dépendante, anti-schistosome. L'activité suppressive a été détectée dans deux pics, mais l'activité optimale a été trouvée dans le second pic, le plus petit, migrant dans la gamme de poids moléculaire inférieure ou égale à 25000 et de préférence comprise entre 18000 et 20000; le premier et plus grand pic pourrait correspondre à une forme agrégée de la molécule.

La charge électrique de la lymphokine inhibitrice plaquettaire PASL a été examinée : l'activité est maximale à un point isoélectrique (Pi) compris entre 4,1 et 4,6, comme le montre la Figure 8 qui montre le pourcentage d'inhibition de la cytotoxicité en fonction du pH. La focalisation du point isoélectrique de la PASL a été réalisée dans une colonne de Sephadex-G75 contenant 5% d'ampholines supports dans une gamme de pH de 3,8 à 10, pendant 16 heures, à une puissance constante de 8 watts. Chaque fraction de

7

gel a été éluée, dialysée pendant une nuit contre du PBS contenant du NaCl, pour éliminer les ampholines, après quoi son activité suppressive a été examinée en évaluant sa cytoxicité plaquettaire IgE-dépendante vis-à-vis des Schistosomules.

Il ressort de ce qui précède que la lymphokine PASL inhibe les fonctions effectrices des plaquettes sanguines, ce qui suggère qu'elle joue un rôle dans la régulation de troubles immunopathologiques dans lesquels le taux d'IgE est augmenté, notamment dans les allergies atopiques, et plus particulièrement dans l'asthme. De ce fait, la PASL constitue une substance immunopharmacologique d'un grand intérêt pour le traitement des troubles allergiques dans lesquels les plaquettes semblent impliquées.

La description qui va suivre se réfère à un exemple de preparation de la lymphokine PASL conforme à la présente invention, qui est donné à titre d'illustration de l'un des objets de la présente invention, et n'a acun caractère limitatif.

Exemple de préparation de lymphokine PASL purifiée

1. Préparation de surnageants de lymphocytes T

Les surnageants de lymphocytes T peuvent être obtenus soit par stimulation mitogènique, soit par stimulation antigénique, comme décrit ci-après.

A. Par stimulation mitogénique
- à partir de cellules humaines,
  $3.10^6$ de cellules T périphériques ou d'amygdales, prélevées chez l'homme, sont incubées dans du milieu RPMI-FCS [c'est-à-dire du milieu RPMI 1640 contenant 5% de sérum de veau foetal inactivé par la chaleur (FCS)] avec de la Concanavaline A (0,01 à 5 $\mu$g/ml) à 37°C dans des plaques de culture comportant des puits à fond plat, pendant 24 heures, dans une atmosphère humidifiée contenant 5% de $CO_2$. Les cellules sont ensuite lavées pour éliminer la Concanavaline A et cultivées pendant 24 heures. Les surnageants de chaque puits sont récupérés, centrifugés à 400xg et filtrés à travers des membranes de porosité 0,22 $\mu$m, après quoi, s'iols ne sont pas utilisés immédiatement, ils sont stockés à - 20°C.
- a partir de cellules murines
  $1.10^6$ lymphocytes de ganglions lymphatiques ont été utilisés et les surnageants préparés comme décrit ci-dessus.

B. Par stimulation antigénique
- la stimulation de cellules T humaines provenant d'un patient atteint d'Echinicoccose a été effectuée dans du milieu RPMI-FCS par addition d'extraits bruts d'Echinococcus granulosus (concentration finale : 5, 40 ou 100) en présence de cellules mononucléaires de sang périphérique (PBMC) autologues irradiées (2000 rads, 2 minutes). Après avoir séjourné 24 heures dans une atmosphère humidifiée, les cellules ont été lavées pour éliminer les extraits précités et elles ont été cultivées pendant 4 jours. Le surnageant de culture a été récupéré, filtré à travers une membrane à 0,22 $\mu$m et stocké à - 20°C s'il n'est pas utilisé immédiatement,
- des cellules T obtenues à partir de ganglions lymphatiques de rats infectés par S. Mansoni (14, 35 ou 56 jours d'infection) ont été incubées dans du milieu RPMI-FCS supplémenté par de la IL2 (Interleukine 2), des cellules thymiques de rat irradiées (APC) et d'antigène de ver adulte de S. mansoni (50 $\mu$g/ml de concentration finale) dans des plaques de culture à puits multiples à fond plat, dans une atmosphère humidifiée contenant 5% de $CO_2$. Cette stimulation a été effectuée tous les 5 jours et le 15ème jour, les surnageants ont été récupérés, centrifugés pour en éliminer toutes cellules contaminantes éventuellement présentes, et filtrés à travers des membranes de 0,22 $\mu$m, avant d'être stockés à - 20°C, si nécessaire. En même temps, la réponse de multiplication des cellules T a été mesurée dans ces conditions, après une impulsion de 16 heures avec 1 $\mu$Ci de $^3$H-Thymidine (1Ci/mmole), la radioactivité incorporée étant déterminée par filtration de la culture à travers des membranes Millipore et comptage des filtres dans un fluide de scintillation liquide, dans un bêta-spectromètre.

2. Purification des surnageants pour en extraire la lymphokine PASL active

2.1. Le surnageant, éventuellement décongelé après stockage, est dialysé en cellule Amicon (membrane UM5) pour en éliminer les sels et les petites molécules.

2.2. Après dialyse, la lymphokine PASL est purifiée par gel-filtration sur une colonne de Biogel-P30 extra fin (granulométrie 400 mesh) en milieu AcOH 1%, à un débit linéaire de 2 cm/heure. Le contrôle des effluents est effectué par lecture à 254 nm et par recherche de l'activité biologique ou immunologique comme décrit plus loin. Les fractions ainsi localisées sont réunies et lyophilisées.

2.3. La protéine (lymphokine PASL) est alors soumise à une chromatographie en phase inverse (CLHP) sur un gel de silice greffé (tel que l'octadécylsilane, par exemple) dont les particules sont de granulométrie contrôlée (5 $\mu$m) et dont les pores sont calibrés (300 Å).

L'élution s'effectue à l'aide d'un gradient de $CH_3CN$-$H_2O$ allant de 1-99 à 60-40, en 3 heures. A ces deux phases est ajouté 1%, d'acide trifluoroacétique pour l'appariement ionique.

La détection est effectuée par enregistrement de densité optique à 215 nm et détermination de l'activité biologique. Les fractions ainsi localisées sont alors lyophilisées et contrôlées : elles sont constituées par la lymphokine PASL.

3. Détection de l'activité biologique

L'activité biologique est détectée par l'aptitude des plaquettes à tuer des larves de schistosome en présence d'IgE spécifiques, et par l'évaluation de la chimioluminescence induite par le métabolisme oxydatif en présence de luminol et de luciférine.

4. Détermination du poids moléculaire de la lymphokine PASL conforme à l'invention

Le surnagant stimulé par la Concanavaline A, dans l'exemple décrit, concentré (2 ml), est filtré à travers une colonne de Sephadex G-75 (1,8 x 38 cm) et élué avec du PBS à un débit de 5 ml/heure. L'activité lymphokinique de chaque fraction est testée comme décrit en 3. ci-dessus.

Pour déterminer le poids moléculaire de la lymphokine, le chromatogramme est calibré avec un kit de calibrage de faible poids moléculaire (fourni par PHARMACIA, UPPSALA, Suède) contenant de la sérum-albumine bovine (BSA, p.m. 67000), de l'ovalbumine (p.m. 43000), du chymotrysinogène A (p.m. 25000) et de la ribonucléase A (p.m. 13700). Le poids moléculaire du polypeptide qui constitue la lymphokine, déterminé comme indiqué ci-dessus, est compris entre 15000 et 20000.

Ainsi que cela ressort de ce qui précède on isole, conformément à la présente invention, une lymphokine qui se distingue des lymphokines identifiées jusqu'à ce jour, aussi bien par ses propriétés biologiques que par ses propriétés physico-chimiques, et qui inhibe les fonctions effectrices des plaquettes, fournissant ainsi une substance immunopharmacologique propre à jouer un rôle important dans la régulation de troubles immunopathologiques dans lesquels le taux des IgE est augmenté, et plus particulièrement dans les allergies atopiques et plus particulièrement encore dans l'asthme.


**Revendications**

**Revendications pour les Etats contractants suivants : BE, CH, DE, FR, GB, IT, LI, LU, NL, SE**

1. Nouvelle lymphokine suppressive de l'activation plaquettaire, caractérisée en ce qu'elle est constituée par un facteur obtenu à partir de cellules T stimulées par la Concanavaline A ou par un antigène, capable d'inhiber la cytotoxicité plaquettaire IgE-dépendante vis-à-vis de jeunes larves de S. mansoni, de diminuer fortement la chimioluminescence de plaquettes sanguines, dans une réaction IgE/anti-IgE, qui est un corrélat de la cytotoxicité antiparasitaire, et d'inhiber l'activation plaquettaire dans des intolérances non-IgE dépendantes, lequel facteur est désigné sous le nom de lymphokine suppressive de l'activité plaquettaire (PASL) et est produit par les lymphocytes T OKT8[+].

2. Lymphokine selon la revendication 1, caractérisée en ce qu'elle est isolée de surnageant de cultures de lymphocytes T OKT8[+] stimulés par la Concanavaline A ou un antigène.

3. Lymphokine selon la revendication 2, caractérisée en ce que dans le cas où elle est isolée de surnageants de cultures de lymphocytes T OKT8[+] stimulés par un antigène, les lymphocytes T ont été stimulés par des antigènes d'Echinococcus granulosus ou de Schistosoma mansoni.

4. Procédé de préparation d'une lymphokine suppressive de l'activité plaquettaire, caractérisé en ce qu'il consiste à stimuler des cellules T d'origine humaine ou murine par la Concanavaline A ou un stimulant mitogènique analogue ou par un antigène pris dans le groupe qui comprend les antigènes de S. mansoni et les antigènes d'Echinococcus granulosus, à cultiver les cellules T stimulées, pendant une durée appropriée, à récupérer les surnageants de culture et à les filtrer, éventuellement après centrifugation, à travers des membranes de dimension de pores de l'ordre de 0,22 $\mu$m, puis à les purifier par gel-filtration et à soumettre la fraction protéique active recueillie, préalablement identifiée par recherche de l'activité biologique ou immunologique et éventuellement lyophilisée, à une chromatographie en phase inverse sur un gel de silice greffé, de granulométrie et de porosité calibrées, suivie d'une élution par un gradient de $CH_3CN$-$H_2O$ contenant 1% d'acide trifluoroacétique, allant de 1-99 à 60-40, la fraction protéique purifiée contenant la PASL étant ensuite avantageusement lyophilisée, après détection par enregistrement de densité optique à 215 nm et détermination de l'activité biologique.

**5.** Procédé selon la revendication 4, caractérisé en ce que l'activité biologique de la fraction protéique active et de la fraction protéique active purifiée contenant la PASL est déterminée par l'obtention de 60% d'inhibition de la cytotoxicité par le surnageant de culture de lymphocytes T stimulés par la Concanavaline A ou par des antigènes d'E. granulosus, vis-à-vis de plaquettes sanguines normales utilisées comme cellules effectrices, incubées avec du sérum de patients atteints de schistosomiase ou avec du sérum de patients allergiques présentant un taux élevé d'IgE circulantes et stimulés par des anti-IgE ou par l'allergène spécifique.

**6.** Nouveau médicament immunomodulateur des manifestations allergiques, caractérisé en ce que son constituant actif est la PASL isolée de surnageants de cultures de lymphocytes T stimulés par un agent mitogène tel que la Concanavaline A ou analogue ou par un antigène approprié tel que notamment un antigène d'E. granulosus ou de S. mansoni, et dont le poids moléculaire est de l'ordre de 15 à 20 kDa et le Pi est de l'ordre de 4,4 à 5,0, laquelle lymphokine possède une structure de liaison spécifique du type d'un récepteur, à la surface des plaquettes sanguines, c'est-à-dire une spécificité d'adsorption sur les plaquettes.

**Revendications pour les Etats contractants suivants : AT, ES, GR**

**1.** Procédé de préparation d'une nouvelle lymphokine suppressive de l'activation plaquettaire, caractérisé en ce que l'on prépare une lymphokine constituée par un facteur obtenu à partir de cellules T stimulées par la Concanavaline A ou par un antigène, capable d'inhiber la cytotoxicité plaquettaire IgE-dépendante vis-à-vis de jeunes larves de S. mansoni, de diminuer fortement la chimio-luminescence de plaquettes sanguines, dans une réaction IgE/anti-IgE, qui est un corrélat de la cytotoxicité anti-parasitaire, et d'inhiber l'activation plaquettaire dans des intolérances non-IgE dépendantes, lequel facteur est désigné sous le nom de lymphokine suppressive de l'activité plaquettaire (PASL) et est produit par les lymphocytes T OKT8$^+$.

**2.** Procédé selon la revendication 1, caractérisé en ce que la lymphokine est isolée de surnageant de cultures de lymphocytes T OKT8$^+$ stimulés par la Concanavaline A ou un antigène.

**3.** Procédé selon la revendication 2, caractérisé en ce que dans le cas où la lymphokine est isolée de surnageants de cultures de lymphocytes T OKT8$^+$ stimulés par un antigène, les lymphocytes T ont été stimulés par des antigènes d'Echinococcus granulosus ou de Schistosoma mansoni.

**4.** Procédé de préparation d'une lymphokine suppressive de l'activité plaquettaire, caractérisé en ce qu'il consiste à stimuler des cellules T d'origine humaine ou murine par la Concanavaline A ou un stimulant mitogènique analogue ou par un antigène pris dans le groupe qui comprend les antigènes de S. mansoni et les antigènes d'Echinococcus granulosus, à cultiver les cellules T stimulées, pendant une durée appropriée, à récupérer les surnageants de culture et à les filtrer, éventuellement après centrifugation, à travers des membranes de dimension de pores de l'ordre de 0,22 $\mu$m,
puis à les purifier par gel-filtration et à soumettre la fraction protéique active recueillie, préalablement identifiée par recherche de l'activité biologique ou immunologique et éventuellement lyophilisée, à une chromatographie en phase inverse sur un gel de silice greffé, de granulométrie et de porosité calibrées, suivie d'une élution par un gradient de $CH_3CN-H_2O$ contenant 1% d'acide trifluoroacétique, allant de 1-99 à 60-40, la fraction protéique purifiée contenant la PASL étant ensuite avantageusement lyophilisée, après détection par enregistrement de densité optique à 215 nm et détermination de l'activité biologique.

**5.** Procédé selon la revendication 4, caractérisé en ce que l'activité biologique de la fraction protéique active et de la fraction protéique active purifiée contenant la PASL est déterminée par l'obtention de 60% d'inhibition de la cytotoxicité par le surnageant de culture de lymphocytes T stimulés par la Concanavaline A ou par des antigènes d'E. granulosus, vis-à-vis de plaquettes sanguines normales utilisées comme cellules effectrices, incubées avec du sérum de patients atteints de schistosomiase ou avec du sérum de patients allergiques présentant un taux élevé d'IgE circulantes et stimulés par des anti-IgE ou par l'allergène spécifique.

6.  Procédé de préparation d'un nouveau médicament immunomodulateur des manifestations allergiques, caractérisé en ce que son constituant actif est la PASL isolée de surnageants de cultures de lymphocytes T stimulés par un agent mitogène tel que la Concanavaline A ou analogue ou par un antigène approprié tel que notamment un antigène d'E. granulosus ou de S. mansoni, et dont le poids moléculaire est de l'ordre de 15 à 20 kDa et le Pi est de l'ordre de 4,4 à 5,0, laquelle lymphokine possède une structure de liaison spécifique du type d'un récepteur, à la surface des plaquettes sanquines, c'est-à-dire une spécificité d'odsorption sur les plaquettes.

**Claims**

**Claims for the following Contracting States : BE, CH, DE, FR, GB, IT, LI, LU, NL, SE**

1.  New platelet-activation suppressive lymphokine, characterised in that it consists of a factor obtained from T cells stimulated with concanavalin A or with an antigen, capable of inhibiting IgE-dependent platelet cytotoxicity with respect to young S. mansoni larvae, of strongly decreasing the chemoluminescence of blood platelets in an IgE/anti-IgE reaction, which is a correlate of antiparasitic cytotoxicity, and of inhibiting platelet activation in non-IgE-dependent intolerance, which factor is designated by the name platelet-activity suppressive lymphokine (PASL) and is produced by T OKT8[+] lymphocytes.

2.  Lymphokine according to Claim 1, characterised in that it is isolated from culture supernatant of T OKT8[+] lymphocytes stimulated with concanavalin A or an antigen.

3.  Lymphokine according to Claim 2, characterised in that, in the case where it is isolated from culture supernatants of T OKT8[+] lymphocytes stimulated with an antigen, the T lymphocytes have been stimulated with Echinococcus granulosus or Schistosoma mansoni antigens.

4.  Method for preparing a platelet-activity suppressive lymphokine, characterised in that it consists in stimulating T cells of human or murine origin with concanavalin A or a similar mitogenic stimulant or with an antigen selected from the group comprising S. mansoni antigens and Echinococcus granulosus antigens, in culturing the stimulated T cells for a suitable period, in recovering the culture supernatants and filtering them, where appropriate after centrifugation, through membranes of pore size of the order of 0.22 $\mu$m, and then in purifying them by gel filtration and subjecting the active protein fraction collected, identified beforehand by investigating the biological or immunological activity and, where appropriate, lyophilised, to reversed-phase chromatography on a grafted silica gel of calibrated particle size and porosity, followed by elution with a $CH_3CN/H_2O$ gradient containing 0.1% of trifluoroacetic acid, ranging from 1:99 to 60:40, the purified protein fraction containing PASL then being advantageously lyophilised after detection by recording optical density at 215 nm and determining the biological activity.

5.  Method according to Claim 4, characterised in that the biological activity of the active protein fraction and of the purified active protein fraction containing PASL is determined by obtaining a 60% inhibition of the cytotoxicity with the culture supernatant of T lymphocytes, stimulated with concanavalin A or with E. granulosus antigens, with respect to normal blood platelets used as effector cells and incubated with serum of patients suffering from schistosomiasis or with serum of allergic patients possessing a high level of circulating IgE, and stimulated with anti-IgE or with the specific allergen.

6.  New medicinal product having an immunomodulatory effect on allergic manifestations, characterised in that its active constituent is PASL isolated from supernatants of cultures of T lymphocytes stimulated with a mitogenic agent such as concanavalin A or the like or with a suitable antigen such as, in particular, an E. granulosus or S. mansoni antigen, and whose molecular weight is of the order of 15 to 20 kDa and Pi is of the order of 4.4 to 5.0, which lymphokine possesses a specific, receptortype binding structure at the surface of blood platelets, that is to say a specificity of adsorption on platelets.

**Claims for the following Contracting States : AT, ES, GR**

1.  Method for preparing a new platelet-activation suppressive lymphokine, characterised in that a lymphokine is prepared, the latter consisting of a factor obtained from T cells stimulated with concanavalin A or with an antigen, capable of inhibiting IgE-dependent platelet cytotoxicity with respect to young S.

mansoni larvae, of strongly decreasing the chemoluminescence of blood platelets in an IgE/anti-IgE reaction, which is a correlate of antiparasitic cytotoxicity, and of inhibiting platelet activation in non-IgE-dependent intolerance, which factor is designated by the name platelet-activity suppressive lymphokine (PASL) and is produced by T OKT8[+] lymphocytes.

2.  Method according to Claim 1, characterised in that the lymphokine is isolated from culture supernatants of T OKT8[+] lymphocytes stimulated with concanavalin A or an antigen.

3.  Method according to Claim 2, characterised in that, in the case where the lymphokine is isolated from culture supernatants of T OKT8[+] lymphocytes stimulated with an antigen, the T lymphocytes have been stimulated with Echinococcus granulosus or Schistosoma mansoni antigens.

4.  Method for preparing a platelet-activity suppressive lymphokine, characterised in that it consists in stimulating T cells of human or murine origin with concanavalin A or a similar mitogenic stimulant or with an antigen selected from the group comprising S. mansoni antigens and Echinococcus granulosus antigens, in culturing the stimulated T cells for a suitable period, in recovering the culture supernatants and filtering them, where appropriate after centrifugation, through membranes of pore size of the order of 0.22 μm, and then in purifying them by gel filtration and subjecting the active protein fraction collected, identified beforehand by investigating the biological or immunological activity and, where appropriate, lyophilised, to reversed-phase chromatography on a grafted silica gel of calibrated particle size and porosity, followed by elution with a $CH_3CN/H_2O$ gradient containing 0.1% of trifluoroacetic acid, ranging from 1:99 to 60:40, the purified protein fraction containing PASL then being advantageously lyophilised after detection by recording optical density at 215 nm and determining the biological activity.

5.  Method according to Claim 4, characterised in that the biological activity of the active protein fraction and of the purified active protein fraction containing PASL is determined by obtaining a 60% inhibition of the cytotoxicity with the culture supernatant of T lymphocytes, stimulated with concanavalin A or with E. granulosus antigens, with respect to normal blood platelets used as effector cells and incubated with serum of patients suffering from schistosomiasis or with serum of allergic patients possessing a high level of circulating IgE, and stimulated with anti-IgE or with the specific allergen.

6.  Method for preparing a new medicinal product having an immunomodulatory effect on allergic manifestations, characterised in that its active constituent is PASL isolated from supernatants of cultures of T lymphocytes stimulated with a mitogenic agent such as concanavalin A or the like or with a suitable antigen such as, in particular, an E. granulosus or S. mansoni antigen, and whose molecular weight is of the order of 15 to 20 kDa and Pi is of the order of 4.4 to 5.0, which lymphokine possesses a specific, receptor-type binding structure at the surface of blood platelets, that is to say a specificity of adsorption on platelets.

**Patentansprüche**
**Patentansprüche für folgende Vertragsstaaten : BE, CH, DE, FR, GB, IT, LI, LU, NL, SE**

1.  Neues, die Aktivierung der Blutplättchen suprimierendes Lymphokin, dadurch **gekennzeichnet,** daß es aus einem Faktor besteht, der, ausgehend von durch Concanavalin A oder durch ein Antigen stimulierten T-Zellen, erhalten worden ist, der in der Lage ist, die IgE-abhängige Zytotoxizität der Blutplättchen gegenüber jungen Larven von S. mansoni zu inhibieren, stark die Chemolumineszenz der Blutplättchen in einer IgE/anti-IgE-Reaktion, die der antiparasitären Zytotoxizität entspricht, zu vermindern und die Aktivierung der Blutplättchen bei nicht IgE-abhängigen Unverträglichkeiten zu inhibieren, wobei der Faktor als die Aktivität der Blutplättchen suprimierendes Lymphokin (PASL) bezeichnet wird und von den T-OKT8[+]-Lymphozyten gebildet wird.

2.  Lymphokin nach Anspruch 1, dadurch **gekennzeichnet,** daß es aus dem Kulturüberstand von durch Concanavalin A oder ein Antigen stimulierten T-OKT8[+]-Lymphozyten isoliert worden ist.

3.  Lymphokin nach Anspruch 2, dadurch **gekennzeichnet,** daß in dem Falle, in dem es aus Kulturüberständen von durch ein Antigen stimulierten T-OKT8[+]-Lymphozyten isoliert worden ist, die T-Lymphozyten durch Antigene von Echinococcus granulosus oder von Schistosoma mansoni stimuliert worden sind.

**4.** Verfahren zur Herstellung eines, die Blutplättchenaktivität suprimierenden Lymphokins, dadurch **gekennzeichnet,** daß man T-Zellen aus Menschen oder Mäusen durch Concanavalin A oder ein analoges mitogenes Stimulanz oder ein Antigen aus der Gruppe, bestehend aus Antigenen von S. mansoni und Antigenen von Echinococcus granulosus, stimuliert, die stimulierten T-Zellen eine geeignete Zeit inkubiert, die Kulturüberstände gewinnt und filtriert, gegebenenfalls nach Zentrifugation durch Membranen mit Porendurchmessern in der Größenordnung von 0,22 $\mu$m, anschließend sie durch Gelfiltration reinigt und die gewonnene aktive Proteinfraktion, die zuvor durch die Analyse auf die biologische oder immunologische Aktivität identifiziert worden ist und, gegebenenfalls, lyophilisiert worden ist, einer Umkehrphasen-Chromatographie auf einem beschichteten Silicagel mit kalibrierter Korngröße und Porosität, mit einer Elution über einen $CH_3CN$-$H_2O$-Gradienten, der 1 % Trifluoressigsäure enthält, der von 1-99 auf 60-40 geht, unterwirft, wobei die gereinigte Proteinfraktion, die das PASL enthält, anschließend vorteilhafterweise lyophilisiert wird, nachdem durch Aufzeichnen der optischen Dichte bei 215 nm und Bestimmen der biologischen Aktivität detektiert worden ist.

**5.** Verfahren nach Anspruch 4, dadurch **gekennzeichnet,** daß die biologische Aktivität der aktiven Proteinfraktion und der aktiven gereinigten Proteinfraktion, die das PASL enthält, durch Erhalt einer 60-%igen Hemmung der Zytotoxizität durch den Kulturüberstand von durch Concanavalin A oder Antigene von E. granulosus stimulierten T-Lymphozyten gegenüber normalen Blutplättchen, die als Effektor-Zellen verwendet wurden und die mit Serum von an Schistosomiase erkrankten Patienten oder mit Serum von allergischen Patienten, die einen erhöhten Spiegel an zirkulierendem IgE zeigen und die durch anti-IgE oder durch ein spezielles Allergen stimuliert worden sind, inkubiert worden sind, bistimmt wird.

**6.** Neues Medikament, das allergische Erscheinungen immunmoduliert, dadurch **gekennzeichnet,** daß sein wirksamer Bestandteil, das PASL ist, das aus den Kulturüberständen von durch ein mitogenes Agenz, wie beispielsweise Concanavalin A oder ein Analogon, oder durch ein geeignetes Antigen, wie insbesondere ein Antigen von E. granulosus oder von S. mansoni, und wovon das Molekulargewicht in der Größenordnung von 15 bis 20 kDa und der Pi-Wert in der Größenordnung von 4,4 bis 5,0 liegt, stimulierten T-Lymphozyten, wobei das Lymphokin eine spezifische Bindungsstruktur vom Typ eines Rezeptors an der Oberfläche der Blutplättchen, d.h. eine Absorptionsspezifität auf den Blutplättchen besitzt.

**Patentansprüche für folgende Vertragsstaaten : AT, ES, GR**

**1.** Verfahren zur Herstellung eines neuen, die Aktivierung der Blutplättchen suprimierenden Lymphokins, dadurch **gekennzeichnet,** daß man ein Lymphokin herstellt, bestehend aus einem Faktor, der, ausgehend von durch Concanavalin A oder durch ein Antigen stimulierten T-Zellen, erhalten worden ist, der in der Lage ist, die IgE-abhängige Zytotoxizität der Blutplättchen gegenüber jungen Larven von S. mansoni zu inhibieren, stark die Chemolumineszenz der Blutplättchen in einer IgE/anti-IgE-Reaktion, die der antiparasitären Zytotoxizität entspricht, zu vermindern und die Aktivierung der Blutplättchen bei nicht IgE-abhängigen Unverträglichkeiten zu inhibieren, wobei der Faktor als die Aktivität der Blutplättchen suprimierendes Lymphokin (PASL) bezeichnet wird und von den T-OKT8$^+$ Lymphozyten gebildet wird.

**2.** Verfahren nach Anspruch 1, dadurch **gekennzeichnet,** daß das Lymphokin aus dem Kulturüberstand von durch Concanavalin A oder ein Antigen stimulierten T-OKT8$^+$-Lymphozyten isoliert wird.

**3.** Verfahren nach Anspruch 2, dadurch **gekennzeichnet,** daß in dem Falle, in dem das Lymphokin aus den Kulturüberständen von durch ein Antigen stimulierten T-OKT8$^+$-Lymphozyten isoliert wird, die T-Lymphozyten durch Antigene von Echinococcus granulosus oder von Schistosoma mansoni simuliert worden sind.

**4.** Verfahren zur Herstellung eines, die Blutplättchenaktivität suprimierenden Lymphokins, dadurch **gekennzeichnet,** daß man T-Zellen aus Menschen oder Mäusen durch Concanavalin A oder ein analoges mitogenes Stimulanz oder ein Antigen aus der Gruppe, bestehend aus Antigenen von S. mansoni und Antigenen von Echinococcus granulosus, stimuliert, die stimulierten T-Zellen eine geeignete Zeit inkubiert, die Kulturüberstände gewinnt und filtriert, gegebenenfalls nach Zentrifugation durch Membranen mit Porendurchmessern in der Größenordnung von 0,22 $\mu$m, anschließend sie durch Gelfiltration reinigt und die gewonnene aktive Proteinfraktion, die zuvor durch die Analyse auf die biologische oder immunologische Aktivität identifiziert worden ist und, gegebenenfalls, lyophilisiert worden ist, einer Umkehrphasen-Chromatographie auf einem beschichteten Silicagel mit kalibrierter Korngröße und Porosität, mit einer Elution über einen $CH_3CN-H_2O$-Gradienten, der 1 % Trifluoressigsäure enthält, der von 1-99 auf 60-40 geht, unterwirft, wobei die gereinigte Proteinfraktion, die das PASL enthält, anschließend vorteilhafterweise lyophilisiert wird, nachdem durch Aufzeichnen der optischen Dichte bei 215 nm und Bestimmen der biologischen Aktivität detektiert worden ist.

5. Verfahren nach Anspruch 4, dadurch **gekennzeichnet,** daß die biologische Aktivität der aktiven Proteinfraktion und der aktiven gereinigten Proteinfraktion, die das PASL enthält, durch Erhalt einer 60-%igen Hemmung der Zytotoxizität durch den Kulturüberstand von durch Concanavalin A oder Antigene von E. granulosus stimulierten T-Lymphozyten gegenüber normalen Blutplättchen, die als Effektor-Zellen verwendet wurden und die mit Serum von an Schistosomiase erkrankten Patienten oder mit Serum von allergischen Patienten, die einen erhöhten Spiegel an zirkulierendem IgE zeigen und die durch anti-IgE oder durch ein spezielles Allergen stimuliert worden sind, inkubiert worden sind, bistimmt wird.

6. Verfahren zur Herstellung eines neuen Medikaments, das ailergische Erscheinungen immunmoduliert, dadurch **gekennzeichnet,** daß sein wirksamer Bestandteil, das PASL ist, das aus den Kulturüberständen von durch ein mitogenes Agenz, wie beispielsweise Concanavalin A oder ein Analogon, oder durch ein geeignetes Antigen, wie insbesondere ein Antigen von E. granulosus oder von S. mansoni, und wovon das Molekulargewicht in der Größenordnung von 15 bis 20 kDa und der Pi-Wert in der Größenordnung von 4,4 bis 5,0 liegt, stimulierten T-Lymphozyten, wobei das Lymphokin eine spezifische Bindungsstruktur vom Typ eines Rezeptors an der Oberfläche der Blutplättchen, d.h. eine Absorptionsspezifität auf den Blutplättchen besitzt.

EP 0 246 967 B1

FIG.1

FIG.2

FIG. 3

FIG. 4

FIG.5

% ACTIVITE SUPPRESSIVE

CON A + CELLULES T

CONA + CELLULES OKT8⁺ OKT4⁻

CON A + CELLULES OKT8⁻ OKT4⁺

FIG.6

% INHIBITION CYTOTOXICITE

TRYPSINE    PROTEINASE E    NEURAMINIDASE

TRAITEMENT    SURNAGEANT

17

## FIG.7

## FIG. 8